# EUROPÄISCHE PATENTSCHRIFT

(11) **EP 1 797 908 B1**
(45) Veröffentlichungstag und Bekanntmachung des Hinweises auf die Patenterteilung: **12.01.2011**
(21) Anmeldenummer: 06124901.7
(22) Anmeldetag: 28.11.2006
(51) Int. Cl.: A61L 27/04, A61L 27/30

(54) **Verfahren zur Herstellung eines metallischen Substrates mit biokompatibler Oberfläche und das damit hergestellte Substrat**
Method for preparing a metallic substrate with biocompatible surface and the substrate obtained thereby
Procédé de préparation d'un substrat métallique ayant une surface biocompatible et le substrat obtenu

(30) Priorität: 29.11.2005 DE 102005058125
(43) Veröffentlichungstag der Anmeldung: 20.06.2007
(73) Patentinhaber: BAM Bundesanstalt für Materialforschung und -prüfung, 12205 Berlin (DE)
(72) Erfinder: Berger, Georg, Dr., OT Zepernick, Panketal 16341 (DE); Ploska, Ute, Dr., 12435, Berlin (DE)
(74) Vertreter: Walter, Wolf-Jürgen

(56) Entgegenhaltungen:
- DE-A1- 3 709 457
- DE-C1- 19 944 970
- US-A- 5 178 901
- US-A- 5 478 237

## Beschreibung

Die Erfindung betrifft ein Verfahren zur Herstellung eines metallischen Substrates mit biokompatibler Oberfläche und das damit hergestellte Substrat.

Aus der DE 199 44 970 C1 ist ein Verfahren bekannt, bei dem ein Implantat oder Substrat aus Titan bzw. einer Ti-Legierung mit einer Calciumsalz-Schmelze behandelt wird, um eine biokompatible Oberfläche zu erhalten. Dabei wurden Schichten von 1-2 µm aus Ca₄Ti₃O₁₀ gebildet.

Aufgabe der Erfindung ist es, bei hohen Festigkeiten dünnere Schichten zu bilden, die zugleich fest haften und dies auch bei anderen Metallen als Titan zu ermöglichen.

Erfindungsgemäß besteht das Verfahren zur Herstellung eines metallischen Substrates mit biokompatibler Oberfläche darin, dass man ein Metall, ausgewählt aus der Gruppe, bestehend aus Titan; Titanlegierungen mit Aluminium, Vanadium, Tantal, Niob, Nickel, Eisen, Molybdän oder Gemische davon; Tantal; Tantallegierungen mit Eisen, Aluminium, Chrom oder Gemische davon; rostfreiem Stahl;
mit einer Schmelze behandelt, bestehend aus Calciumnitrat und einer weiteren Komponente, wobei die weitere Komponente ausgewählt ist aus der Gruppe, bestehend aus
einem Sauerstoffsalz von Natrium, Kalium, Lithium, Magnesium und Gemischen davon,
und die Behandlung bei einer Temperatur im Bereich von 180 bis 480°C erfolgt für einen Zeitraum von 0,1 bis 12 Stunden, wobei der Calciumgehalt der Schmelze wenigstens 20 Masse-% beträgt, bezogen auf das Gesamtgewicht der Schmelze.

Als bevorzugte Titanlegierung wird eine Legierung aus Titan mit Al und/oder V und/oder Nb und/oder Ni und/oder Fe und/oder Mo eingesetzt.

Als bevorzugte Tantallegierung wird eine Legierung aus Tantal mit Eisen und/oder Aluminium und/oder Chrom eingesetzt.

Als weitere Komponente ist ein Nitrat oder Sulfat von Ca, K, Li oder Na bevorzugt, wobei NaNO₃ vorteilhaft ist. Besonders bevorzugt ist ein Gemisch aus Calciumnitrat und den weiteren Komponenten NaNO₃, KNO₃, LiNO₃ in Masse-% Ca(NO₃)₂ 20 bis 95; NaNO₃ 1 - 50; KNO₃ 1 -50; LiNO₃ 0 - 20.

In dem Verfahren liegt die Temperatur vorteilhaft im Bereich von 200 bis 480°C, vorzugsweise 250 bis 460°C. Bei diesen niedrigen Temperaturen findet keine Vergrößerung der Schichtdicke statt, sondern sie bleibt etwa konstant und führt damit insgesamt zu dünneren Schichten als bisher bekannt.

Die Behandlungszeit liegt vorteilhaft im Bereich von 0,5 bis 8h, vorzugsweise 0,5 bis 4h, insbesondere 0,5-2 Stunden.

Es wurde gefunden, dass unter Zusatz eines oder mehrerer Sauerstoffsalze von Natrium, Kalium, Lithium oder Magnesium bei wesentlich niedrigeren Temperaturen als bisher gebräuchlich sehr dünne Schichten von 5-1600 nm, vorteilhaft 10-1000 nm, insbesondere 10-800 nm, speziell 20-450 nm erhalten werden können. Solche Schichten können mit dem erfindungsgemäßen Verfahren nicht nur auf Titan oder Titanlegierungen, sondern auch auf anderen für biokompatible Anwendung geeigneten Stählen aufgetragen werden, z.B. auf rostfreien Stählen, oder auf Tantalstählen. Bei Titan bildet sich unter den o.g. Bedingungen eine Schicht aus CaTiO₃.

Eine Temperung wie bei dem bekannten Verfahren ist nicht erforderlich.

Unter "Sauerstoffsalze" werden verstanden Nitrate, Nitrite, Sulfate oder bestimmte Salze organischer Säuren wie Oxalate oder Hydroxysuccinate.

Das erfindungsgemäße Verfahren führt durch die deutliche Temperaturabsenkung und die dabei gebildeten dünneren Schichten zu keinerlei Festigkeitsverlusten. Es wurde gefunden, dass z.B. die für unbehandelte Titanlegierungen festzustellenden Schwingfestigkeiten von 500-600 MPa bei einer Schwingzahl N von 10⁷-10^{5,5} für solche gemäß DE 19944970 behandelte Titanlegierungen mit 400 MPa bei N=10⁶ und 600 bei N=10⁵ erheblich niedriger liegen. Die erfindungsgemäß behandelten Materialien (Titan, Titanlegierungen, Tantal, Tantallegierung, rostfreier Stahl) bleiben jedoch im Bereich der Werte für die jeweiligen unbehandelte Materialien und sind damit dem Stand der Technik klar überlegen. Die Schwingfestigkeit wurde nach dem entsprechenden Test für ungekerbte Rundproben gemäß DIN 50113-A durchgeführt.

Besonders vorteilhaft bei Titan oder Titanlegierungen, wie Ti-Al-V, Ti-Ta-Nb, Ti-Ni, Ti-Al-Nb, gegebenenfalls mit Mo und/oder Fe, ist die Ausbildung einer calciumhaltigen Reaktionsschicht wegen der dabei auftretenden vorteilhaften Wirkungen der Verbesserung des direkten, bindegewebsfreien Knochenkontaktes. Die oxidische Schicht kann mit dem Calcium chemische Verbindungen, zum Beispiel zu Calciumtitanaten, eingehen.

Es ist weiterhin vorteilhaft, dass durch das erfindungsgemäße Verfahren das Absorptionsverhalten von Proteinen, an den Reaktionsschichten geändert wird. Dadurch ist ein schnelleres biologisch angepassteres Einwachsen eines Substrates möglich.

Durch das erfindungsgemäße Verfahren wird die Schmelze nicht so schnell bzw. kaum zersetzt, d.h. die Bildung nitroser Gase wird deutlich verzögert. Weiterhin besteht ein größeres Temperaturintervall für die Erzeugung der Reaktionsschicht auf dem Metallsubstrat unter Berücksichtigung der entsprechenden Behandlungszeiten.

Ein weiterer Gegenstand der Erfindung ist ein metallisches Substrat mit biokompatibler Oberfläche, bei dem auf dem Substrat, ausgewählt aus der Gruppe, bestehend aus Titan; Titanlegierungen mit Aluminium, Vanadium, Tantal, Niob, Nickel, Eisen, Molybdän oder Gemische davon; Tantal; Tantallegierungen mit Eisen, Aluminium, Chrom oder Gemische davon; rostfreiem Stahl;
eine Reaktionsschicht ausgebildet ist, bestehend aus einer dem Substrat zugewandten inneren oxidischen Schicht und einer äußeren Ca-haltigen Schicht, und die Schichtdicke insgesamt im Bereich von 10 bis unter 1600 nm liegt. Die äußere Ca-haltige Schicht kann auf ihrer nach außen zugewandten Oberfläche Einlagerungen von Na-, K-, Li-, Mg-Atomen oder Gemischen davon aufweisen, wodurch auch die Löslichkeit/Stabilität des Interface Implantat/Knochen nachhaltig beeinflusst wird sowie der in die Körperflüssigkeit wechselnde Ionenfluss hinsichtlich Qualität und Quantität verändert ist.

Dabei ist die Ca-haltige Schicht eine in die oxidische Schicht integrierte Reaktionsschicht, hervorgegangen aus der Reaktion eines geschmolzenen Ca-Salzes mit der oxidischen Oberflächenschicht. In der Regel wird die oxidische Oberflächenschicht auf dem Metallgrundkörper im Prozess durch die erhöhte Temperatur und unter dem Einfluss der Salzschmelze verstärkt, d.h. die messbare Dicke dieser Schicht nimmt zu. Damit handelt es sich nicht um eine aufgetragene Schicht, die auf der Oberfläche liegt, sondern sie ist in die Metalloberfläche hinein integriert (Reaktionsschicht). In diese Schicht können weitere Elemente bis zu einer bestimmten Tiefe der Schicht integriert sein. Vorteilhaft wird bei dem erfindungsgemäßen Substrat die Schicht deutlich dünner, d.h. sie liegt im Bereich von 10-1500 nm, insbesondere 10-800 nm, speziell 20-450 nm.

Ein weiteres Merkmal des Substrates besteht darin, dass dessen Schwingfestigkeit im Bereich der Schwingfestigkeit des unbehandelten Substrates liegt.

Bevorzugt ist ein metallisches Substrat, das aus Titan oder einer titanhaltigen Legierung besteht, die Ca-haltige Schicht aus Calciumtitanat als Hauptbestandteil besteht und auf deren nach außen zugewandten Oberfläche Einlagerungen von Na-, K-, Li-, Mg-Atomen oder Gemischen davon in der Ca-haltigen Schicht vorhanden sein können. Ähnliche Einschlüsse können auch in den anderen Metallen oder Metalllegierungen enthalten sein.

Die Erfindung soll nachstehend durch Beispiele näher erläutert werden. Die darin genannten Prozente sind Masseprozente (Ma%).

### Beispiel 1

Eine entfettete, gewaschene und getrocknete Probe aus TiA16V4 wird in einer Salzschmelze aus Calciumnitrat-Tetrahydrat

(74Ma%) und Natriumnitrat (26Ma%) für 4h bei 450°C gelagert. Anschließend wird sie mit heißem Wasser im Ultraschallbad (10min) und mit verdünnter Salzsäure (1 Teil HCl konz. und 9 Teile Wasser - konz. = konzentrierte Salzsäure, als konz. Salzsäure wird 37%-ige Salzsäure bezeichnet) ebenfalls im Ultraschallbad (5min) gereinigt, mit voll entsalztem Wasser und dann mit absolutem Alkohol gewaschen und getrocknet. Mittels Röntgendiffraktometrie an dünnen Schichten (TF-XRD) wurde auf der Oberfläche die Bildung von Calciumtitanat nachgewiesen. Schichtdicke der oxidischen und Ca-haltigen Schicht 190 nm, gemessen mit AUGER-Elektronenspektroskopie.

### Beispiel 2

Eine entfettete, gewaschene und getrocknete Probe aus NiTi wird in einer Salzschmelze aus Calciumnitrat-Tetrahydrat (74Ma%) und Natriumnitrat(26Ma%)für 2h bei 480°C gelagert. Anschließend wird sie mit heißem Wasser im Ultraschallbad (10min) und mit verdünnter Salzsäure (1 Teil HCl (konz.) und 9 Teile Wasser - auch in den folgenden Beispielen) ebenfalls im Ultraschallbad (5min) gereinigt, mit voll entsalztem Wasser und dann mit absolutem Alkohol gewaschen und getrocknet. Mittels TF-XRD wurde auf der Oberfläche die Bildung von Calciumtitanat nachgewiesen.

### Beispiel 3

Eine entfettete, gewaschene und getrocknete Probe aus TiAl6V4 wird in einer Salzschmelze aus Calciumnitrat (63Ma%), Natriumnitrat(17Ma%)und Kaliumnitrat(23Ma%)für 2h bei 400°C gelagert. Anschließend wird sie mit heißem Wasser im Ultraschallbad (10min) und mit verdünnter Salzsäure (1 Teil HCl + 9 Teile Wasser) ebenfalls im Ultraschallbad (5min) gereinigt, mit voll entsalztem Wasser und dann mit absolutem Alkohol gewaschen und getrocknet. Schichtdicke (wie Bsp. 1) 150 nm gemessen mit AUGER-Elektronenspektroskopie. Weiterhin wurde mittels AUGER-Elektronenspektrometrie in der Substratoberfläche Ca bis zu einer Tiefe von 15nm nachgewiesen.

### Beispiel 4

Eine entfettete, gewaschene und getrocknete Probe aus hochreinem cp-Titan wird in einer Salzschmelze aus Calciumnitrat (70Ma%), Natriumnitrat(12Ma%), Kaliumnitrat (12Ma%) und Lithiumnitrat (6Ma%) für 2h min bei 250°C gelagert. Anschließend wird sie mit heißem Wasser im Ultraschallbad (10min) und mit verdünnter Salzsäure (1 Teil HCl + 9 Teile Wasser) ebenfalls im Ultraschallbad (5min) gereinigt, mit voll entsalztem Wasser und dann mit absolutem Alkohol gewaschen und getrocknet. Schichtdicke (wie Bsp. 1) 20 nm, gemessen mit AUGER-Elektronenspektroskopie.

### Beispiel 5

Eine entfettete, gewaschene und getrocknete Probe aus Implantatstahl wird in einer Salzschmelze aus Calciumnitrat-Tetrahydrat (74Ma%) und Natriumnitrat(26Ma%) für 2h bei 450°C gelagert. Anschließend wird sie mit heißem Wasser im Ultraschallbad (10min) und mit verdünnter Salzsäure (1 Teil HCl und 9 Teile Wasser) ebenfalls im Ultraschallbad (5min) gereinigt, mit voll entsalztem Wasser und dann mit absolutem Alkohol gewaschen und getrocknet.

Mittels Elektronenspektrometrie (ESCA) wurden Calcium und Natrium in der oberflächennahen Schicht nachgewiesen. Schichtdicke der oxidischen und Ca-haltigen Schicht 290 nm, gemessen mit AUGER-Elektronenspektroskopie.

### Beispiel 6

Eine entfettete, gewaschene und getrocknete Probe aus Tantalblech wird in einer Salzschmelze aus Calciumnitrat (70Ma%)und Natriumnitrat (12Ma%) für 4h min bei 480°C gelagert. Anschließend wird sie mit heißem Wasser im Ultraschallbad (10min) und mit verdünnter Salzsäure (1 Teil HCl + 9 Teile Wasser) ebenfalls im Ultraschallbad (5min) gereinigt, mit voll entsalztem Wasser und dann mit absolutem Alkohol gewaschen und getrocknet.

Mittels TF-XRD wurde auf der Oberfläche die Bildung von Calciumtantalat nachgewiesen.
Schichtdicke der oxidischen und Ca-haltigen Schicht 220 nm, gemessen mit AUGER-Elektronenspektroskopie.

### Beispiel 7

Eine entfettete, gewaschene und getrocknete Probe aus TiA16V4 wird in einer Salzschmelze aus Calciumnitrat (63Ma%), Natriumnitrat(17Ma%)und Kaliumnitrat(23Ma%)für 25min bei 250°C gelagert. Anschließend wird sie mit heißem Wasser im Ultraschallbad (10min) und mit verdünnter Salzsäure (1 Teil HCl +9 Teile Wasser) ebenfalls im Ultraschallbad (5min) gereinigt, mit voll entsalztem Wasser und dann mit absolutem Alkohol gewaschen und getrocknet.

Mittels AUGER-Elektronenspektrometrie wurde in der Substratoberfläche Ca bis zu einer Tiefe von 8nm nachgewiesen.

## Patentansprüche

1. Verfahren zur Herstellung eines metallischen Substrates mit biokompatibler Oberfläche,
**dadurch gekennzeichnet, dass** man ein Metall, ausgewählt aus der Gruppe, bestehend aus
Titan; Titanlegierungen mit Aluminium, Vanadium, Tantal, Niob, Nickel, Eisen, Molybdän oder Gemische davon; Tantal; Tantallegierungen mit Eisen, Aluminium, Chrom oder Gemische davon und rostfreiem Stahl;
mit einer Schmelze behandelt, bestehend aus Calciumnitrat und einer weiteren Komponente, wobei die weitere Komponente ausgewählt ist aus der Gruppe, bestehend aus
einem Sauerstoffsalz von Natrium, Kalium, Lithium, Magnesium und Gemischen davon,
und die Behandlung bei einer Temperatur im Bereich von 180 bis 480°C erfolgt für einen Zeitraum von 0,1 bis 12 Stunden, wobei der Calciumgehalt der Schmelze wenigstens 20 Masse-% beträgt, bezogen auf das Gesamtgewicht der Schmelze.

2. Verfahren nach Anspruch 1, **dadurch gekennzeichnet, dass** als Titanlegierung eine Legierung aus Titan mit Aluminium und Vanadin, oder eine Legierung aus Titan mit Niob, oder eine Legierung aus Titan mit Nickel, oder eine Legierung aus Titan mit Niob, Tantal und Zirkonium eingesetzt wird.

3. Verfahren nach Anspruch 1, **dadurch gekennzeichnet, dass** als Tantallegierung eine Legierung aus Tantal mit einem oder mehreren der Elemente Eisen, Aluminium und Chrom eingesetzt wird.

4. Verfahren nach Anspruch 1, **dadurch gekennzeichnet, dass** eine Schmelze aus Calciumnitrat und als weitere Komponente NaNO₃ eingesetzt wird, insbesondere 20 bis 95 Ma% Ca(NO₃)₂ und 1 - 50 Ma% NaNO₃ eingesetzt wird.

5. Verfahren nach Anspruch 1, **dadurch gekennzeichnet, dass** eine Schmelze aus Calciumnitrat und als weitere Komponente NaNO₃, KNO₃ und LiNO₃ eingesetzt wird, insbesondere in Masse-% Ca(NO₃)₂ 20 bis 95; NaNO₃ 1 - 50; KNO₃ 1 -50; LiNO₃ 0 - 20.

6. Verfahren nach Anspruch 1, **dadurch gekennzeichnet, dass** die Temperatur im Bereich von 200 bis 480 °C liegt, vorzugsweise 200 bis 460°C.

7. Verfahren nach Anspruch 1, **dadurch gekennzeichnet, dass** die Zeit im Bereich von 0,5 bis 8 Stunden liegt, vorzugsweise 0,5 bis 4 Stunden.

8. Durch das Verfahren nach Anspruch 1 herstellbares metallisches Substrat mit biokompatibler Oberfläche,
**dadurch gekennzeichnet, dass** auf dem Substrat, ausgewählt aus der Gruppe, bestehend aus Titan; Titanlegierungen mit Aluminium, Vanadium, Tantal, Niob, Nickel, Eisen, Molybdän oder Gemische davon; Tantal; Tantallegierungen mit Eisen, Aluminium, Chrom oder Gemische davon und rostfreiem Stahl;
eine Schicht ausgebildet ist, bestehend aus einer dem Substrat zugewandten inneren oxidischen Schicht und einer äußeren Ca-haltigen Schicht, und die Schichtdicke insgesamt im Bereich von 10 bis unter 1600nm liegt.

9. Metallisches Substrat nach Anspruch 8, **dadurch gekennzeichnet, dass** die äußere Ca-haltige Schicht auf ihrer nach außen zugewandten Oberfläche Einlagerungen von Na-, K-, Li-, Mg-Atomen oder Gemischen davon enthält.

10. Metallisches Substrat mit biokompatibler Oberfläche nach Anspruch 8, **dadurch gekennzeichnet, dass** die Ca-haltige Schicht eine in die oxidische Schicht integrierte Reaktionsschicht ist, hervorgegangen aus der Reaktion eines geschmolzenen Ca-Salzes mit der oxidischen Schicht.

11. Metallisches Substrat mit biokompatibler Oberfläche nach Anspruch 8, **dadurch gekennzeichnet, dass**
das Substrat aus Tantal oder einer tantalhaltigen Legierung besteht und eine Ca-haltige Reaktionsschicht enthält, wobei die oxidische Sicht mit dem Calcium eine chemische Verbindung eingeht.

12. Metallisches Substrat mit biokompatibler Oberfläche nach Anspruch 9, **dadurch gekennzeichnet, dass** die Einlagerungen an Natrium, Kalium, Lithium oder Gemischen davon in der äußeren Ca-haltigen Schicht bis zu einer Tiefe von 20 % der Schichtdicke der Ca-haltigen Schicht vorhanden sind.

13. Metallisches Substrat nach Anspruch 8, **dadurch gekennzeichnet, dass** be**i** einem Substrat, ausgewählt aus der Gruppe, bestehend aus Titanlegierungen, Tantal, Tantallegierungen und Implantatstählen wenigstens in der äußeren Ca-haltigen Reaktionsschicht die Konzentration der weiteren Legierungsmetalle Aluminium, Vanadium, Niob, Nickel, Eisen, Molybdän, Chrom und Gemische davon um bis zu 20 Masse-%, bezogen auf die Masse des Legierungsmetalls, niedriger liegt als im Inneren des Substrats.

14. Metallisches Substrat nach Anspruch 8, **dadurch gekennzeichnet, dass** die Schichtdicke im Bereich von 5-1600 nm, vorteilhaft 10-1500 nm, insbesondere 10-800 nm, speziell 20-450 nm liegt.

15. Metallisches Substrat nach Anspruch 8, **dadurch gekennzeichnet**, das die Schwingfestigkeit des Substrats im gleichen Bereich der Schwingfestigkeit liegt wie die eines unbehandelten Substrats bei gleicher Schwingzahl N.

## Claims

1. A method for the production of a metallic substrate with biocompatible surface,
which comprises a metal selected from the group consisting of
titanium; titanium alloys with aluminum, vanadium, tantalum, niobium, nickel, iron, molybdenum or mixtures thereof; tantalum; tantalum alloys with iron, aluminum, chromium or mixtures thereof, and stainless steel is treated with a melt consisting of calcium nitrate and an additional component, said additional component being selected from the group consisting of
an oxygen salt of sodium, potassium, lithium, magnesium and mixtures thereof;
said treatment being effected at a temperature ranging from 180 to 480 °C for a period of from 0.1 to 12 hours, the calcium content of the melt being at least 20% by weight, relative to the total weight of the melt.

2. The method according to claim 1, wherein an alloy of titanium with aluminum and vanadium or an alloy of titanium with niobium or an alloy of titanium with nickel or an alloy of titanium with niobium, tantalum and zirconium is used as titanium alloy.

3. The method according to claim 1, wherein an alloy of tantalum with one or more of the elements iron, aluminum and chromium is used as tantalum alloy.

4. The method according to claim 1, wherein a melt of calcium nitrate and NaNO₃ as additional component is used, especially 20 to 95 wt. -% Ca(NO₃)₂ and 1 to 50 wt.-% NaNO₃ is used.

5. The method according to claim 1, wherein a melt of calcium nitrate and NaNO₃, KNO₃ and LiNO₃ as additional component is used, especially in wt.-% Ca(NO₃)₂ 20 to 95; NaNO₃ 1 to 50; KNO₃ 1 to 50; LiNO₃ 0 to 20.

6. The method according to claim 1, wherein the temperature is in the range of 200 to 480°C, preferably 200 to 460°C.

7. The method according to claim 1, wherein the time is in the range of 0.5 to 8 hours, preferably 0.5 to 4 hours.

8. A metallic substrate producible by the method according to claim 1, having a biocompatible surface,
wherein the substrate, being selected from the group consisting of titanium; titanium alloys with aluminum, vanadium, tantalum, niobium, nickel, iron, molybdenum or mixtures thereof; tantalum; tantalum alloys with iron, aluminum, chromium or mixtures thereof, and stainless steel;
has a layer formed thereon, consisting of an inner oxide layer facing the substrate and an outer Ca-containing layer, the overall layer thickness ranging from 10 to below 1600 nm.

9. The metallic substrate according to claim 8, wherein the outer Ca-containing layer has inclusions of Na, K, Li, Mg atoms or mixtures thereof on its outwardly facing surface.

10. The metallic substrate with biocompatible surface according to claim 8, wherein the Ca-containing layer is a reaction layer integrated in the oxide layer and produced from the reaction of a fused Ca salt with the oxide layer.

11. The metallic substrate with biocompatible surface according to claim 8, wherein the substrate consists of tantalum or of a tantalum-containing alloy and includes a Ca-containing reaction layer, said oxide layer producing a chemical compound with said calcium.

12. The metallic substrate with biocompatible surface according to claim 9, wherein the inclusions of sodium, potassium, lithium or mixtures thereof in the outer Ca-containing layer are present down to a depth of 20% of the layer thickness of the Ca-containing layer.

13. The metallic substrate according to claim 8, wherein in a substrate selected from the group consisting of titanium alloys, tantalum, tantalum alloys, and implant steels, at least in the outer Ca-containing reaction layer, the concentration of the additional alloy metals aluminum, vanadium, niobium, nickel, iron, molybdenum, chromium and mixtures thereof is lower than that inside the substrate by up to 20% by weight, relative to the weight of the alloy metal.

14. The metallic substrate according to claim 8, wherein the layer thickness ranges from 5 to 1600 nm, advantageously from 10 to 1500 nm, particularly from 10 to 800 nm, and specifically from 20 to 450 nm.

15. The metallic substrate according to claim 8, wherein the fatigue strength of the substrate is in the same fatigue strength range as that of an untreated substrate at equal number of vibrations N.

## Revendications

1. Procédé de préparation d'un substrat métallique ayant une surface biocompatible,
**caractérisé en ce que** l'on traite un métal, choisi parmi le groupe consistant en
le titane ; les alliages du titane avec l'aluminium, le vanadium, le tantale, le niobium, le nickel, le fer, le molybdène ou leurs mélanges ; le tantale ; les alliages du tantale avec le fer, l'aluminium, le chrome ou leurs mélanges et l'acier inoxydable ;
avec une masse fondue consistant en le nitrate de calcium et un autre composant, où l'autre composant est choisi parmi le groupe consistant en un sel oxygéné du sodium, du potassium, du lithium, du magnésium et leurs mélanges,
et on réalise un traitement à une température située dans l'intervalle allant de 180 à 480°C pendant une période allant de 0,1 à 12 heures, où la teneur en calcium de la masse fondue se situe à au moins 20% en masse, sur base du poids total de la masse fondue.

2. Procédé selon la revendication 1, **caractérisé en ce que** l'on met en oeuvre comme alliage du titane, un alliage de titane avec l'aluminium et le vanadium, ou un alliage du titane avec le niobium, ou un alliage du titane avec le nickel, ou un alliage du titane avec le niobium, le tantale et le zirconium.

3. Procédé selon la revendication 1, **caractérisé en ce que** l'on met en oeuvre comme alliage du tantale, un alliage du tantale avec un ou plusieurs des éléments fer, aluminium et chrome.

4. Procédé selon la revendication 1, **caractérisé en ce que** l'on met en oeuvre une masse fondue de nitrate de calcium et comme autre composant, NaNO₃, en particulier 20 à 95% en poids de Ca(NO₃)₂ et 1-50% en poids de NaNO₃.

5. Procédé selon la revendication 1, **caractérisé en ce que** l'on met en oeuvre une masse fondue de nitrate de calcium et comme autre composant, NaNO₃, KNO₃ et LiNO₃, en particulier en % en poids, Ca(NO₃)₂ 20 à 95 ; NaNO₃ 1-50 ; KNO₃ 1-50 ; LiNO₃ 0-20.

6. Procédé selon la revendication 1, **caractérisé en ce que** la température se situe dans l'intervalle allant de 200 à 480°C, de préférence de 200 à 460°C.

7. Procédé selon la revendication 1, **caractérisé en ce que** la durée se situe dans l'intervalle allant de 0,5 à 8 heures, de préférence de 0,5 à 4 heures.

8. Substrat métallique ayant une surface biocompatible, pouvant être préparé par le procédé selon la revendication 1, **caractérisé en ce que** l'on forme sur le substrat, choisi parmi le groupe consistant en le titane ; les alliages du titane avec l'aluminium, le vanadium, le tantale, le niobium, le nickel, le fer, le molybdène ou leurs mélanges ; le tantale ; les alliages du tantale avec le fer, l'aluminium, le chrome ou leurs mélanges et l'acier inoxydable ; une couche, consistant en une couche oxyde interne orientée vers le substrat et une couche externe contenant du Ca, l'épaisseur des couches se situant au total, dans la plage allant de 10 à moins de 1600 nm.

9. Substrat métallique selon la revendication 8, **caractérisé en ce que** la couche extérieure contenant du Ca contient sur sa surface orientée vers l'extérieur, des insertions d'atomes de Na, K, Li, Mg ou leurs mélanges.

10. Substrat métallique ayant une surface biocompatible selon la revendication 8, **caractérisé en ce que** la couche contenant le Ca est une couche réactionnelle intégrée dans la couche oxyde, engendrée par la réaction d'un sel de Ca fondu avec la couche oxyde.

11. Substrat métallique ayant une surface biocompatible selon la revendication 8, **caractérisé en ce que** le substrat consiste en du tantale ou un alliage contenant du tantale et contient une couche réactionnelle contenant du Ca, où la couche oxyde donne un composé chimique avec le calcium.

12. Substrat métallique ayant une surface biocompatible selon la revendication 9, **caractérisé en ce que** les insertions de sodium, de potassium, de lithium ou de leurs mélanges sont présentes dans la couche extérieure contenant du Ca jusqu'à une profondeur de 20% de l'épaisseur de la couche contenant le Ca.

13. Substrat métallique selon la revendication 8, **caractérisé en ce que** pour un substrat choisi dans le groupe consistant en les alliages du titane, le tantale, les alliages du tantale et les aciers pour implant, au moins dans la couche extérieure de réaction contenant du Ca, la concentration des autres métaux d'alliage, aluminium, vanadium, niobium, fer, molybdène, chrome et leurs mélanges se situe à jusqu'à 20% en poids, sur base du poids des métaux d'alliage, et est inférieure à celle dans l'intérieur du substrat.

14. Substrat métallique selon la revendication 8, **caractérisé en ce que** l'épaisseur de couche se situe dans l'intervalle allant de 5 à 1600 nm, avec avantage de 10 à 1500 nm, en particulier de 10 à 800 nm, spécialement de 20 à 450 nm.

15. Substrat métallique selon la revendication 8, **caractérisé en ce que** la résistance à la fatigue du substrat se situe dans la même plage que la résistance à la fatigue du substrat non traité à même indice de fatigue N.
